# EUROPEAN PATENT SPECIFICATION

(11) **EP 1 484 021 B1**
(45) Date of publication and mention of the grant of the patent: **18.05.2011**
(21) Application number: 04013050.2
(22) Date of filing: 02.06.2004
(51) Int. Cl.: A61B 17/04

(54) **Medical Suturing Instrument**
Medizinische Wundnähvorrichtung
Dispositif medical de suture

(30) Priority: 06.06.2003 JP 2003162008
(43) Date of publication of application: 08.12.2004
(73) Proprietor: Olympus Corporation, Shibuya-ku, Tokyo (JP)
(72) Inventor: Mikkaichi, Takayasu, Fuchu-shi, Tokyo (JP); Suzuki, Keita, Kokubunji-shi, Tokyo (JP)
(74) Representative: von Hellfeld, Axel

(56) References cited:
- US-A- 6 056 760
- US-B1- 6 287 317
- US-B1- 6 500 184

## Description

### BACKGROUND OF THE INVENTION

### 1. Field of the Invention

The present invention relates to a suturing instrument that is introduced into a body cavity via the channel of an endoscope.

This application is based on Japanese Patent Application No. 2003-162008.

### 2. Description of the Related Art

Methods are conventionally known for introducing procedure instruments into a body cavity in order to perform a necessary procedure. As an example of a device employed in procedures of this sort, there is a device for suturing tissue in which a pushing member is employed to push an anchoring member out from a catheter, this anchoring member being attached to suture material that is housed inside a puncture needle provided to the end of the catheter (see Japanese Patent No. 31 342 88 (paragraphs 0007 and 0010, Figure 4)), for example). The surgical instrument for intracardiac suturing disclosed in Japanese Patent No. 31 342 88 (paragraphs 0007 and 0010, Figure 4) is designed such that the puncture catheter is inserted in a sheath in a manner so that it can be freely put into and taken out from the sheath, and heparinized physiologic saline can be infused into the puncture catheter from a lateral infusion tube.

When the biological tissue to be sutured iies near other biological tissue, such as when suturing bowel or other tubular-shaped organs, it is possible that the puncture needle which penetrates the target tissue could inadvertently penetrate or otherwise injury other nearby biological tissue. Accordingly, this procedure has been problematic with respect to the extreme care that must be exercised during the operation.

### SUMMARY OF THE INVENTION

The present invention resolves the aforementioned problem, and has as its objective the provision of a suturing instrument that penetrates just the targeted area with surety and enables an anchoring member to be retained.

The present invention provides a suturing instrument according to claim 1. Advantageous and preferred features of the suturing instrument of the invention are recited in dependent claims 2 and 3.

The aspect of the present invention is a suturing instrument having an inserted portion, which is to be introduced into a body cavity and is equipped with a hollow puncture needle having an opening formed at one end, and an operating portion, which is for operating a pushing member that is passed through the inserted portion, wherein the pushing member is employed to push an anchoring member that is attached to suture material out from the end of the puncture needle, and the suture material is to be passed through the biological tissue while the anchoring member is retained in the biological tissue; characterized in that this suturing instrument is provided with a flow introducing portion for introducing flow discharged from the puncture needle, and a flow path that is disposed so as to enable the anchoring member to be pushed out of the puncture needle and permits flow inside the puncture needle.

The suturing instrument according to this invention enables flow introduced from the flow introducing portion to be supplied to the puncture needle via the flow path when penetrating the suture target with the puncture needle. Thus, the puncture needle is passed through the biological tissue as flow is being discharged, so that the organ, etc. can be insufflated during penetration of the needle.

An embodiment of the present invention is characterized in that, in the suturing instrument of the first aspect of the present invention, the anchoring member is formed to have a cylindrical shape and permits flow through the space inside.

The suturing instrument according to this invention ensures the flow path by permitting flow inside the anchoring member when the anchoring member is housed inside the puncture needle, and enables the flow to be discharged from the end of the puncture needle.

A second embodiment of the present invention is characterized in that, the operating portion has a main body portion, which is equipped with the flow introducing portion, and a needle operating portion, that is moveable with respect to the aforementioned main body portion and can advance and retract the puncture needle, and in that the needle operating portion is provided with a communicating hole for communicating between the flow introducing portion and the flow path, and flow sealing members for cutting off the flow from the flow introducing portion to the flow path once the puncture needle has been moved a set amount.

In an embodiment of this invention, the flow sealing members are attached at positions that stop the flow supply once the puncture needle has been moved a specific amount only. Thus, flow is supplied until the puncture needle has been moved this specific amount. Once the specific amount is exceeded, the communicating path between the flow introducing portion and the flow path is blocked off.

### BRIEF DESCRIPTION OF THE DRAWINGS

Figure 1 is a schematic view of an endoscope treatment device that includes the suturing instrument according to the present invention.
Figure 2 is a cross-sectional view showing the structure of the suturing instrument.
Figures 3A to 3E are views typically showing the steps through retention of the anchoring member in a luminal organ.
Figures 4A and 4B are perspective views showing a modification of the anchoring member and the pushing member.
Figure 5A is a cross-sectional view and Figure 5B is a perspective view showing a modification of the anchoring member and the pushing member.
Figure 6A is a cross-sectional view and Figure 6B is a perspective view showing a modification of the anchoring member and the pushing member.
Figures 7A and 7B are perspective views showing a modification of the anchoring member and the pushing member.
Figure 8 is a cross-sectional view showing the structure of the suturing instrument.
Figure 9 is a cross-sectional view showing the structure of the suturing instrument.
Figures 10A and 10B are perspective views showing a modification of the anchoring member and the pushing member.

### PREFERRED EMBODIMENTS OF THE INVENTION

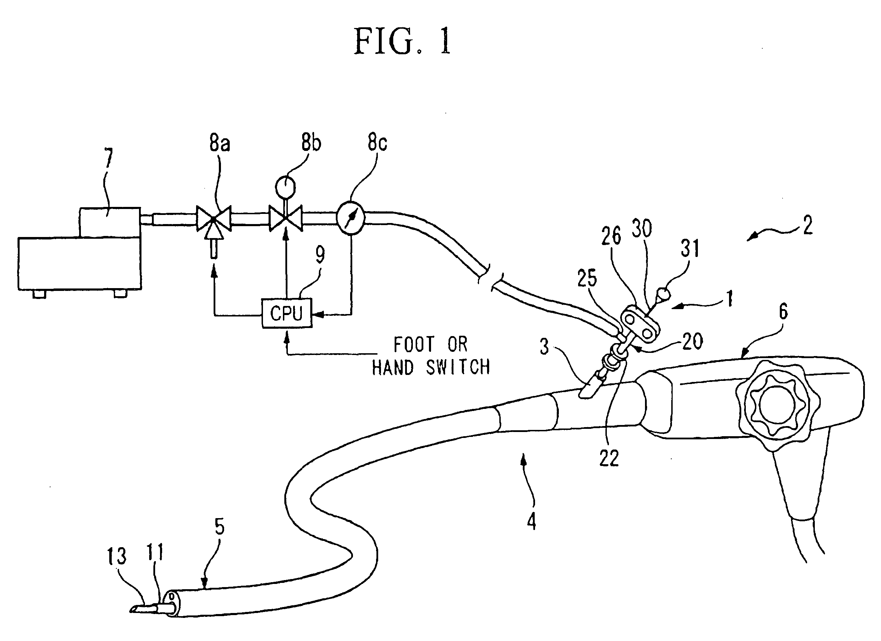

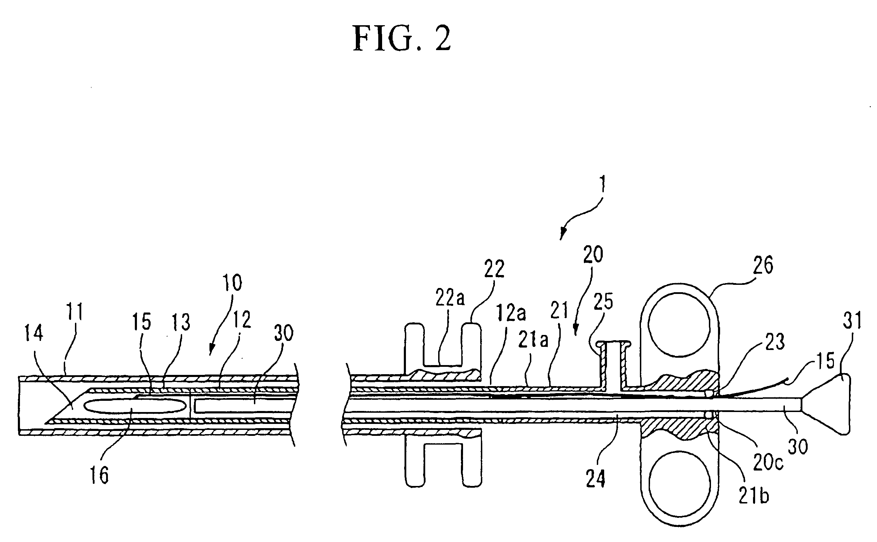

A first embodiment of the present invention will now be explained in detail with reference to the figures. Figure 1 is a schematic view of an endoscope treatment device that includes the suturing instrument according to the present embodiment. Figure 2 is a cross-sectional view showing the structure of the suturing instrument.

As shown in Figures 1 and 2, the present embodiment's suturing instrument 1 is employed by insertion into the forceps channel 3 of endoscope treatment device 2. Endoscope treatment device 2 consists of an endoscope 4 into which suturing instrument 1 is inserted, and an air pump 7 which is the flow supplying means for supplying a gas such as air, i.e., the flow, to suturing instrument 1. Endoscope 4 consists of introduced portion 5 that is introduced into the body cavity and which has a forceps channel 3, illumination and a camera lens, etc. at one end thereof, and an operating portion 6 for operating introduced portion 5 from outside the body cavity.

As shown in Figure 2, suturing instrument 1 has a flexible inserted portion 10 that is inserted into a body cavity, and a hand operated portion 20 which the surgeon operates from outside the body, and is designed so as to permit insertion and passage of a pushing member 30 internally.

Inserted portion 10 has an outer sheath 11, which is a flexible tube, an inner sheath 12 that is housed inside outer sheath 11 in a manner to permit its advance or withdrawal, and a puncture needle 13 that is attached to the end of inner sheath 12. The end of puncture needle 13 is beveled at an acute angle to form a sharp point, thus facilitating its penetration into biological tissue. Puncture needle 13 has a hollow form in which the pointed end thereof has an opening 14. An anchoring member 16, which is attached to suture material 15, is housed internally. The pushing member 30, which is the pusher for sending anchoring member 16 out from the opening 14 of puncture needle 13, is passed from the hand operated portion 20 side to puncture needle 13 and inner sheath 12. Specific clearances are provided between inner sheath 12/puncture needle 13 and pushing member 30, and between puncture needle 13 and anchoring member 16, respectively. These clearances are for enabling the passage of flow, which will be explained later.

Hand operated portion 20 has an operating member 21 that is connected to inner sheath 12; a holding member 22 that is connected to outer sheath 11 and is provided with a knob in which a groove 22a is formed on which the surgeon's fingers rest, and a knob 31 for gripping pushing member 30.

Operating member 21 is in the form of a cylinder with a base, and is fixed in place so that its open end 21a communicates with the open end 12a of inner sheath 12 of inserted portion 10. Further, an opening 20c for passing pushing member 30 is formed to end 21b, which corresponds to the base portion of operating member 21. The diameter of opening 20c is smaller than the inner diameter of the cylindrical portion of operating member 21 and, when used in combination with a packing 23, maintains an air-tight state inside operating member 21 while still permitting sliding movement of pushing member 30. A gas port 25 is attached to the lateral surface of the cylindrical portion of operating member 21, this gas port 25 being the flow introducing portion that is employed when introducing gas supplied from air pump 7 shown in Figure 1 to flow path 24 which is formed by the space within operating member 21. Note that a handle 26 in which rings are formed for the surgeon's fingers to rest is present at end 21b that projects out from the cylindrical portion.

One end of pushing member 30 is pulled out from operating member 21 and a gripping knob 31 is attached thereto. The other end of pushing member 30 extends to the end portion of inner sheath 12 and to puncture needle 13. This pushing member 30 is held so as to permit sliding in opening 20c of operating member 21 and on packing 23. Pushing member 30 can be pulled out from or pushed into operating member 21. When pushing member 30 is pushed into operating member 21, the end thereof applies a pushing force so that anchoring member 16 is pushed out from puncture needle 13.

Anchoring member 16 consists of a circular cylindrically shaped member. One end of suture material 15 for suturing biological tissue is anchored near a center point along the longitudinal direction of anchoring member 16. During suturing, anchoring member 16 comes into contact with the biological tissue in the wide area allong its longitudinal direction, and is anchored in the biological tissue, thus preventing suture material 15 from being pulling out from the biological tissue. As a result of this type of shape and function, anchoring member 16 is sometimes referred to as a T-anchor or T-bar. Note that in Figure 2, suture material 15 extends from the inside of puncture needle 13 and passes through inner sheath 12 to reach hand operated portion 20, with the trailing end of the suture material pulled to the outside of suturing instrument 1. However, it is also acceptable to pull suture material 15 through the opening 14 at the point of puncture needle 13, pass it between outer sheath 11 and inner sheath 12 and pull it to the outside from hand operated portion 20.

The gas relaying mechanism for supplying gas to gas port 25 will now be explained using Figure 1. This gas relaying mechanism has an air pump 7 that suctions up air and then discharges it at a specific pressure. Electromagnetic valve 8a for selecting between relay and exhaust, a flow adjusting valve 8b for adjusting flow of the gas being relayed, and a flow meter 8c for monitoring the flow quantity of gas actually supplied, are connected in this order by tubing on the discharge side of air pump 7. Necessary commands are carried out by a CPU (central processing unit) 9. CPU 9 carries out switching of electromagnetic valve 8a and adjustment of the opening of flow adjusting valve 8b while receiving command signals to start or stop gas relay from a foot or hand switch, and while monitoring flow meter 8c. Note that flow meter 8c and gas port 25 are also connected with tubing.

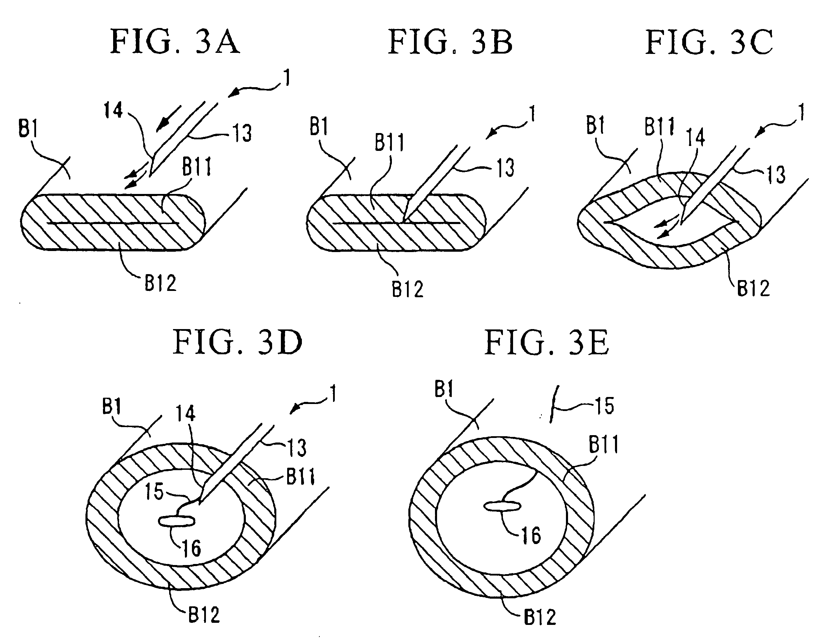

Next, Figures 1 through 3 will be used to explain the sequence for suturing tissue such as an internal organ, using this suturing instrument 1 and taking as an example the case where puncturing a luminal organ such as the bowel. Note that Figure 3 is a view typically showing the sequence through retention of the anchoring member in the luminal organ. Luminal organ B1 shown in cross-section in Figure 3 is collapsed such that biological tissue B11 which is superficial and biological tissue B12 which is deep as seen from the perspective of puncture needle 13 are in contact with one another.

First, flexible endoscope 4 shown in Figure 1 and inserted portion 10 of suturing instrument 1 shown in Figure 2 are introduced into the body cavity. At this time, operating member 21 of hand operated portion 20 is pulled out from holding member 22 by a specific amount only, so that puncture needle 13 is housed inside outer sheath 11. One anchoring member 16 is housed inside puncture needle 13 and pushing member 30 is retracted to a waiting position where it does not apply a pushing force on this anchoring member 16.

Operating member 21 is then pushed forward from this state toward holding member 22 by a specific amount, thereby exposing puncture needle 13 from outer sheath 11. As shown in Figure 3A, puncture needle 13 is moved into a position facing the luminal organ B1 on which suturing is to be performed. A foot or hand switch, not shown in the figures, is turned ON, and gas discharged by air pump 7 shown in Figure 1 is supplied to gas port 25. The gas supplied to gas port 25 in this way is introduced to inner sheath 12 of inserted portion 10 via flow path 24 in operating member 21 shown in Figure 2. The gas passes through the clearance between pushing member 30 and inner sheath 12 and through the clearance between the inner wall of puncture needle 13 and pushing member 30/anchoring member 16, and is discharged from the opening 14 at the end of puncture needle 13 shown in Figure 3A.

When operating member 21 is pushed in, puncture needle 13 penetrates superficial biological tissue B11 of luminal organ B1 while discharging gas from the end thereof (Figure 3B). When the end of puncture needle 13 passes through biological tissue B11, gas is introduced so as to separate the area between biological tissue B11 and biological tissue B12, and, as shown in Figure 3C, insufflate luminal organ B1. Once it has been determined that a specific quantity of gas has been supplied and luminal organ B1 has been insufflated to the extent necessary for the procedure, the foot or hand switch (not shown in the figures) is turned OFF and the supply of gas to gas port 25 is halted. As a result, luminal organ B1 is insufflated so that an air cavity forms inside the organ. Thus, even if puncture needle 13 is pushed forward, it does not puncture deep biological tissue B12. Note that in the typical needle puncture carried out without discharging air, even if superficial biological tissue B11 is penetrated, luminal organ B1 is collapsed, so that biological tissue 11 and biological tissue 12 adhere together or are extremely close to one another. Thus, puncture needle 13 can inadvertently puncture through to deep biological tissue B12.

With luminal organ B1 in an insufflated state following discharge of gas, knob 31 at the end of pushing member 30 in Figure 2 is gripped and is pushed toward operating member 21. As a result, the end of pushing member 30 applies a pushing force on anchoring member 16, and anchoring member 16 is pushed out from opening 14 of puncture needle 13 (Figure 3D). Once it has been confirmed that anchoring member 16 has been pushed out, pushing forward of pushing member 30 is halted, and operating member 21 is withdrawn from holding member 22. Since puncture needle 13 and inner sheath 12 which is connected to operating member 21 are relatively withdrawn, puncture needle 13 is pulled out from biological tissue B11 of luminal organ B1. Anchoring member 16, which at this point in time has already been pushed out and released from puncture needle 13, is retained inside luminal organ B1 as shown in Figure 3E. Suture material 15 which is attached to anchoring member 16 passes through biological tissue B11 from which puncture needle 13 has been pulled out. Even if suture material 15 becomes caught on another procedure instrument, anchoring member 16 has an anchoring effect, so that suture material 15 is not pulled free from luminal organ B1. Next, suture material 15 is tied to another piece of suture material, etc. that has been passed through another organ or biological tissue.

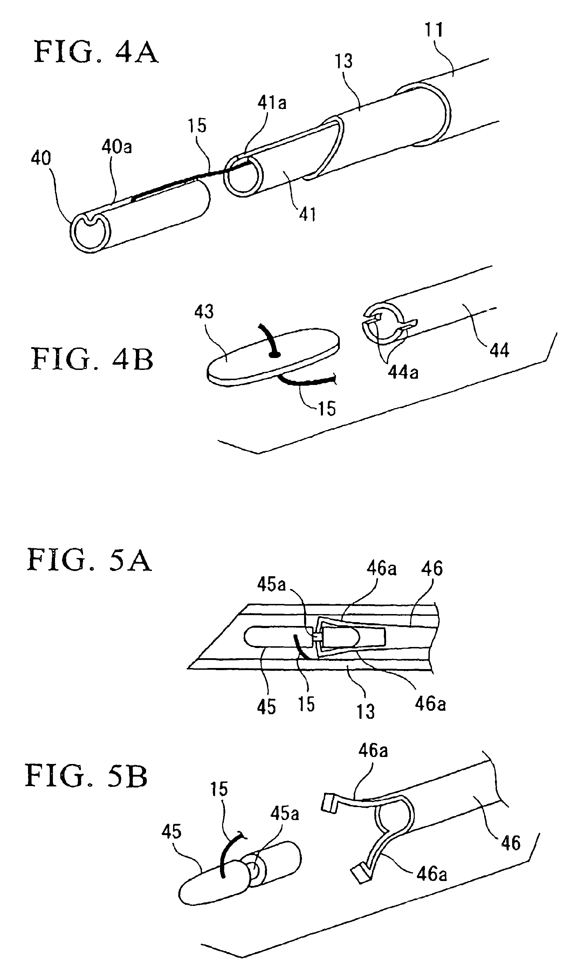

Note that when preventing anchoring member 16 from falling out of puncture needle 13 by bringing anchoring member 16 into contact with the inner wall of puncture needle 13 in a manner to permit sliding movement, a clearance that will permit gas flow can be ensured by providing an anchoring member 16 that is elliptical in cross-section, making the long axis thereof roughly equal to the inner diameter of puncture needle 13 and making the short axis thereof smaller than the inner diameter of puncture needle 13. Further, in the case where the outer diameter of anchoring member 16 is made roughly equal to the inner diameter of puncture needle 13, then it is acceptable to form a groove along the longtidinal direction of anchoring member 16. In the case of an anchoring member 16 having a diameter that is smaller than the inner diameter of puncture needle 13, it is acceptable to bend this anchoring member 16 in the longitudinal direction. Other modifications of anchoring member 16 and its pushing member 30 will be explained below using Figures 4 through 7.

Anchoring member 40 in Figure 4A has a shape wherein a part of the periphery of a hollow pipe having an outer diameter that is roughly equal to the inner diameter of puncture needle 13 is indented, and suture material 15 is attached near the center of this indentation 40a. Pushing member 41 is also formed of a hollow member that has an outer diameter that is roughly equal to the inner diameter of puncture needle 13. In an anchoring member 40 and pushing member 41 of this type, these hollow areas can be employed to permit flow of gas.

The anchoring member 43 shown in Figure 4B consists of a flat plate having a width that is roughly equal to the inner diameter of puncture needle 13. Suture material 15 is attached at the center part thereof. Pushing member 44 for this anchoring member 43 is in the shape of a pipe in which two slits 44a are formed at the end thereof for applying a pressing force on anchoring member 43 with surety. In this case, the hollow area in pushing member 44 and the clearance between anchoring member 43 and the inner wall of puncture needle 13 can be used to permit the flow of gas.

The anchoring member 45 shown in Figures 5A and 5B has a smaller outer diameter than the inner diameter of puncture needle 13, and suture material 15 is attached in the center area along the longitudinal direction thereof. A reduced diameter portion 45a in which the outer diameter has been decreased, is formed in between the end and the position of attachment of suture material 15 along the longitudinal direction. Pushing member 46 has two claws 46a at the end of a hollow pipe for gripping reduced diameter portion 45a of anchoring member 45. These claws 46a are capable of elastic deformity. As shown in Figure 5A, when inside puncture needle 13, claws 46a are pressed against the inner wall of puncture needle 13 and closed, gripping reduced diameter portion 45a of anchoring member 45. On the other hand, when claws 46a exit puncture needle 13, they open as shown in Figure 5B and release their hold on anchoring member 45. For this reason, gas flow, etc. does not cause anchoring member 45 which is housed inside puncture needle 13 to fall free from puncture needle 13. When pushing member 46 is used to push anchoring member 45 out from puncture needle 13, however, anchoring member 45 can easily be released. In this case as well, the hollow portion of pushing member 46 and the clearance between the inner wall of puncture needle 13 and anchoring member 45 can be used to permit flow of gas.

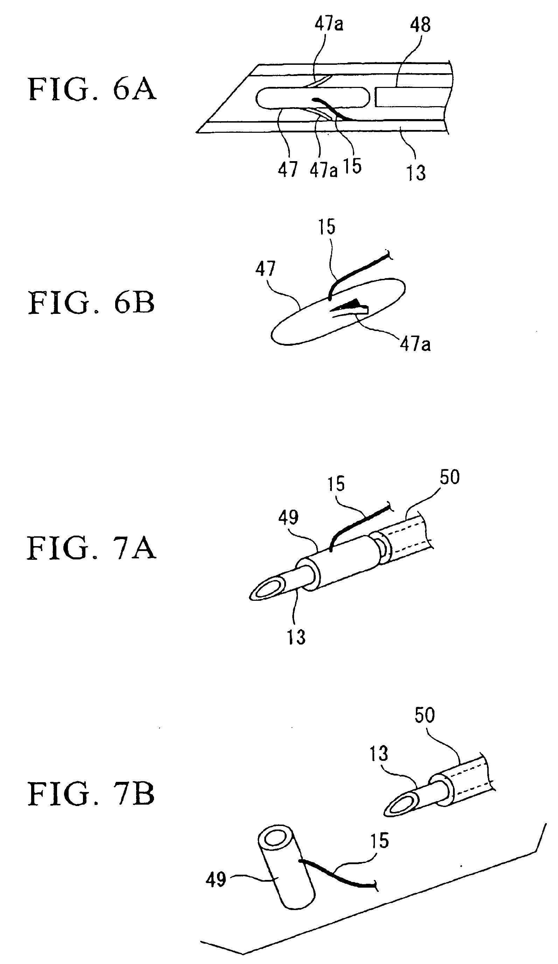

Anchoring member 47 shown in Figures 6A and 6B has a smaller outer diameter than the inner diameter of puncture needle 13, and suture material 15 is attached at the center along the longitudinal direction thereof. Portions of anchoring member 47 are bent, to form stoppers 47a that come into contact with the inner wall of puncture needle 13. Stoppers 47a have the thickest diameter of all portions of anchoring member 47, and that thickness is larger than the inner diameter of puncture needle 13. Accordingly, when anchoring member 47 is housed in puncture needle 13 by pushing back stoppers 47a, movement of anchoring member 47 is restricted by these stoppers 47a. A pushing member 48 suitable for this type of anchoring member 47 is a solid rod that forms a specific clearance with the inner diameter of puncture needle 13. In this case, the clearance between anchoring member 47 and pushing member 48 and the inner wall of puncture needle 13 can be used to permit flow of gas.

The anchoring member 49 shown in Figure 7A consists of a pipe having an inner diameter that is on par with the outer diameter of puncture needle 13, and is employed by attaching to the outside of puncture needle 13. Suture material 15 is attached near the center along the longitudinal direction of anchoring member 49. A pipe attached to the outside of puncture needle 13 may be cited as a pushing member 50 suitable for this type of anchoring member 49. When retaining this anchoring member 49, pushing member 50 is sent along the outside wall of puncture needle 13 as shown in Figure 7B, to push out anchoring member 49 from the end of puncture needle 13. Note that the inside of puncture needle 13 can be employed for the flow of gas.

Next, a second embodiment according to the present invention will be explained in detail with reference to the figures.

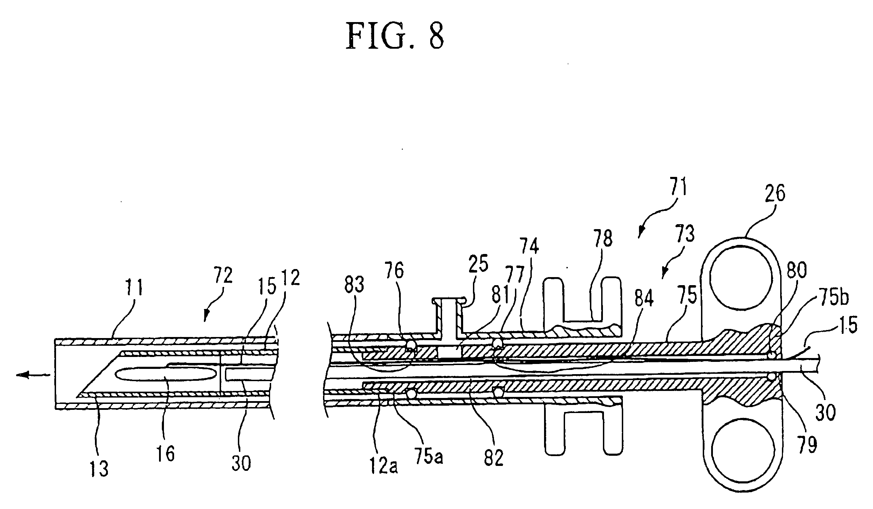

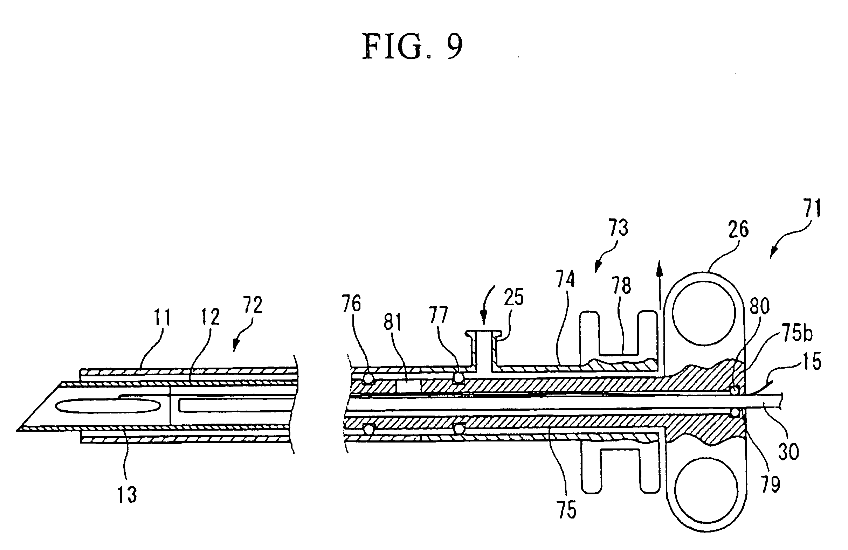

Figures 8 and 9 are cross-sectional views showing the structure of the suturing instrument in this embodiment. Note that components that are identical to those of the first embodiment are denoted with the same numeric symbol and a detailed description thereof has been omitted,

Suturing instrument 71 shown in Figure 8 is employed by being inserted into the forceps channel of an endoscope treatment device, and has an inserted portion 72 that is introduced in a body cavity and a hand operated portion 73 that is operated by the surgeon.

Inserted portion 72 has an outer sheath 11 and an inner sheath 12 that is inserted inside outer sheath 11. Puncture needle 13 is attached to the end of inner sheath 12. Anchoring member 16 is housed inside puncture needle 13. Pushing member 30 which pushes out anchoring member 16 is inserted from the hand operated portion 73 side. Note that anchoring members 40, 43, 45, 47 or 49 shown in Figures 4 through 7 of the first embodiment are also acceptably employed for anchoring member 16.

Hand operated portion 73 is designed as follows. Namely, a needle operating portion 75, that is connected to inner sheath 12 and advances and withdraws puncture needle 13, is attached with a specific amount of clearance inside the operating main body portion 74, which is connected to outer sheath 11, and.flow sealing members 76,77 for adjusting gas relay are interposed between operating main body portion 74 and needle operating portion 75.

Operating main body portion 74 is cylindrical in shape and has a grooved part 78 formed to its outer peripheral surface on which the surgeon rests his fingers. A gas port 25 is provided to operating main body portion 74 for introducing the gas, i.e., the flow, supplied from the air pump, to operating main body portion 74.

Needle operating portion 75 is in the form of a cylinder with a bottom, and is fixed in place so that its open end 75a communicates with the open end 12a of inner sheath 12 of inserted portion 72. An opening 79 through which pushing member 30 passes is formed in end 75b that corresponds to the bottom portion of needle operating portion 75. The diameter of this opening 79 is smaller than the inner diameter of the cylindrical portion of needle operating potion 75, and, when packing 80 is also employed, will permit sliding movement of pushing member 30 while maintaining an airtight state. An air hole 81 is provided to the lateral surface of the cylindrical portion of needle operating portion 75 for guiding gas that is introduced into operating main body portion 74 from gas port 25 into needle operating portion 75. By guiding gas introduced via air hole 81 through flow path 82 that is formed by the space inside needle operating portion 75, gas is supplied to inner sheath 12 and puncture needle 13 which communicate with this flow path 82. Note that a handle 26 in which rings are formed for the surgeon's fingers to rest is provided to end 75b, projecting out from the cylindrical portion of needle operating portion 75.

Two grooves (first groove 83, second groove 84) are formed along the outer periphery of needle operating portion 75 so as to lie on either side of air hole 81. These grooves 83,84 are employed for positioning of and fixing in place flow sealing members 76,77. First groove 83 is formed closer to the inner sheath 12 than air hole 81. Second groove 84 is closer to handle 26 than air hole 81, and is formed in a position that complies with the amount of pushing on puncture needle 13, which will be explained below.

Flow sealing members 76,77, which are interposed between operating main body portion 74 and needle operating portion 75, consist of first packing 76, which is mounted in first groove 83, and second packing 77, which is mounted in second groove 84. First packing 76 and second packing 77 are ringshaped sealing members, such as O-rings, and are in close relationship to grooves 83,84 of needle operating portion 75 and the inner wall of operating main body portion 74 without any interval of spacing therebetween. When needle operating portion 75 is pulled out from operating main body portion 74, i.e., when gas port 25 is positioned between the two packings 76,77, as shown in Figure 8, an airtight state is maintained in the space partitioned by the inner wall of operating main body portion 74, the outer wall of needle operating portion 75 and packing 76,77, and gas introduced into this space is prevented from flowing from anywhere other than air hole 81. As shown in Figure 9, when needle operating portion 75 is pushed into operating main body portion 74 by just a specific amount, and second packing 77 moves beyond the position of attachment of gas port 25 toward inserted portion 72, second packing 77 interrupts the flow of gas from gas port 25 to air hole 81.

Next, an explanation will be made of a suturing procedure employing this suturing instrument 71.

First, suturing instrument 71 is introduced into a body cavity using the channel of a flexible endoscope, and gas from an air pump is supplied to gas port 25 by manipulating a foot or hand switch not shown in the figures. The gas supplied at this time to gas port 25 is introduced to the space that is partitioned by the inner wall of operating main body portion 74, the outer wall of needle operating portion 75 and packing 76,77, and flows from air hole 81 to flow path 82 inside needle operating portion 75. The gas then passes through inner sheath 12 which communicates with flow path 82, and is discharged from opening 14 of puncture needle 13.

When, under these circumstances, handle 26 is gripped and used to push in needle operating portion 75, puncture needle 13 punctures the biological tissue which is to be sutured. Packing 76,77 slide along the inner wall of operating main body portion 74 as needle operating portion 75 is moved. Gas will continue to be supplied from air hole 81 to puncture needle 13 until second packing 77 reaches the position where gas port 25 is installed. As a result, even if needle operating portion 75 and packing 76,77 are being moved, gas continues to be discharged from puncture needle 13.

When needle operating portion 75 is pushed further in, puncture needle 13 penetrates the biological tissue. Since gas from puncture needle 13 is being discharged at this time, when the biological tissue punctured by puncture needle 13 is part of an organ, that organ will insufflate with the gas.

Discharge of gas ceases when puncture needle 13 has completely penetrated the biological tissue. This is because, at that time, needle operating portion 75 has been pushed in to the position shown in Figure 9, and second packing 77, which is pushed in together with needle operating portion 75, moves beyond the installed position of gas port 25 and further toward the biological tissue. Gas introduced from gas port 25 does not flow from air hole 81, but rather is exhausted to the outside via the space between operating main body portion 74 and needle operating portion 75. Anchoring member 16 inside puncture needle 13 is then pushed out by pushing in pushing member 30, and suture material 15 passes through the biological tissue while anchoring member 16 is retained. Suture material 15 is then tied to another suture material, etc., after withdrawing puncture needle 13.

Because this suturing instrument 71 penetrates the biological tissue as gas is being discharged from the end of puncture needle 13 in this way, in the case where suturing an organ, etc., penetration with the needle can be performed as the organ is being insufflated. The amount by which puncture needle 13 is moved when its end is passed through the biological tissue by just the amount that is sufficient to push out anchoring member 16, i.e., the amount by which needle operating portion 75 is pushed in, is investigated in advance, and that position, or a point preceding that position, is set as the position at which the second packing 77 passes by gas port 25 and interrupts the flow of gas to puncture needle 13. Thus, discharge of gas can be stopped so that over-insufflation of the organ following penetration of the needle does not occur. The changeover when halting the discharge of gas is designed to be carried out by packing 76,77 which move accompanying pushing in of puncture needle 13. Thus, gas discharge can be halted at an appropriate timing with surety. First packing 76 performs the function of stopping the discharge of gas from between the outer sheath 11 and inner sheath 12 into the body cavity. However, it is also acceptable to use just second packing 77 for the flow sealing member.

Note that the present invention is not limited to the embodiments described above, but rather has a wide variety of applications.

For example, while the flow discharged from the end of puncture needle 13 may be air or another gas, a liquid such as physiologic saline is also acceptable.

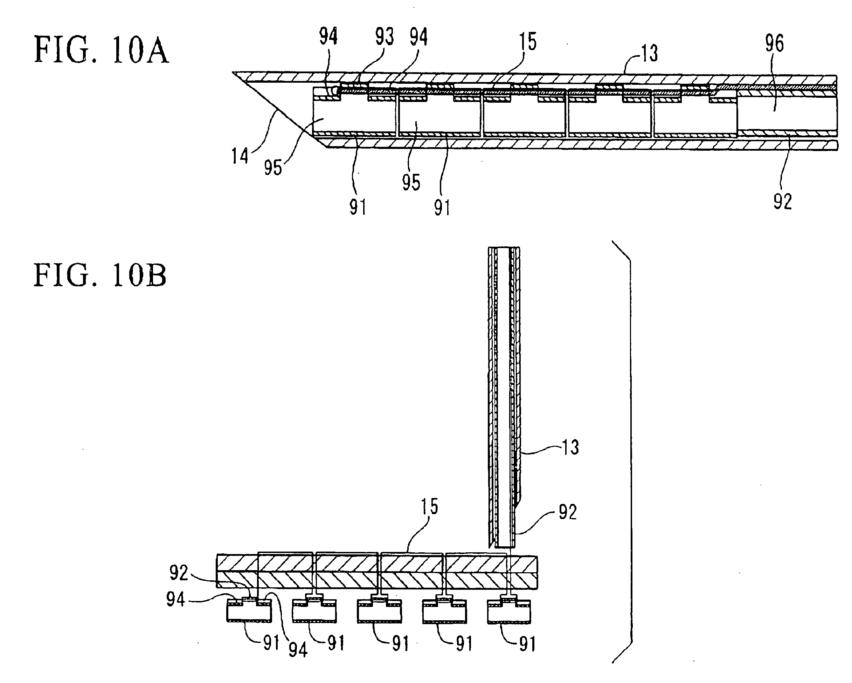

Further, as shown in Figure 10A, a plurality of anchoring members 91 may be housed inside puncture needle 13. Each anchoring member 91 has a cylindrical shape, and parts of the periphery are indented to leave remaining a center portion along the longitudinal direction which is the direction of movement of pushing member 92, so that an interval of space for inserting suture material 15 is formed between unprocessed center portion 93 and indented portion 94. Two adjacent anchoring members 91 are connected by suture material 15 that is passed through their respective space intervals, and are housed inside puncture needle 13. Since anchoring members 91 are housed in a state such that the penetrating holes 95 formed by their cylindrical shape are communicating with one another, gas sent from gas port 25 (see Figure 2) can be discharged from cpening 14 at the end of puncture needle 13 via penetrating holes 95. A pipe-shaped pushing member having a penetrating hole 96 through which gas can flow internally is acceptably employed as a pushing member 92 suitable for this type of anchoring member 91 having a penetrating hole 95. The introduction of gas to pushing member 92 can be realized by providing holes that communicate with flow paths 24,82 of operating main body 21,74.

When performing a suturing procedure using anchoring members 91 that are connected and housed in this way, pushing member 92 is pushed in using hand operated portion 20,73. This pushing member 92 applies a pushing force on the anchoring member 91 that is housed the most rear position, causing the adjacent anchoring member 91 to be pushed toward opening 14 of puncture needle 13. The other anchoring members 91 are pushed toward opening 14 of puncture needle 13 by being by other anchoring members 91 positioned on the pushing member 92 side, and are pushed toward opening 14 of puncture needle 13. Then, in order from the anchoring member 91 that is closest to opening 14 of puncture needle 13, they are pushed inside the organ from puncture needle 13. For example, as Figure 10B typically shows, when suturing two biological tissues, anchoring member 91 is retained in the second biological tissue which puncture needle 13 has punctured. When suture material 15 is pulled in this state, the unprocessed portion 92 into which suture material 15 is inserted is pulled against the biological tissue. Thus, it is possible to ensure a large area of contact between biological tissue and anchoring member 91. Similarly, the remaining anchoring members 91 can be used to achieve a large area of contact between two biological tissues with a specific spacing interval between.

As explained above, the invention according to the first aspect of the invention discharges a flow and enables the puncture needle to be passed through the biological tissue while the organ is being insufflated. Thus, it is possible to carry out a suturing procedure in which only the suture target is penetrated with surety.

The invention according to the second aspect of the present invention ensures the flow path when the anchoring member is housed inside the puncture needle. Thus, flow can be discharged while the needle is penetrating the biological tissue.

In the invention, flow is supplied until the puncture needle has been moved a specific amount. Once the specific amount is exceeded, the communicating path between the flow introducing portion and the flow path is blocked off. Thus, the organ, etc. can be insufflated in a suitable manner.

## Claims

1. A suturing instrument (1) that has an inserted portion (10), which is to be introduced into a body cavity and is equipped with a hollow puncture needle (13) having an opening formed at one end; and an operating portion (21), which is for operating a pushing member (30) that is passed through said inserted portion; wherein said pushing member is employed to push an anchoring member (16) that is attached to a suture material (15) out from the end of said puncture needle, and said suture material is to be passed through a biological tissue, while said anchoring member is retained in said biological tissue; said operating portion comprising:
a flow introducing portion (25) for introducing flow to be discharged from said puncture needle; and **characterized by**
a flow path (24) which permits this flow inside said puncture needle when said anchoring member is enabled to be pushed out of said puncture needle.

2. A suturing instrument according to claim 1, wherein said anchoring member is formed to have a cylindrical shape.

3. A suturing instrument according to claim 1, wherein said operating portion (20) has a main body portion (74), which is equipped with said flow introducing portion, and a needle operating portion (76) that is moveable with respect to said main body portion and can advance and retract said puncture needle; and wherein said needle operating portion is provided with a communicating hole for communicating between said flow introducing portion and said flow path, and flow sealing members for cutting off the flow from said flow introducing portion to said flow path once said puncture needle has been moved a set amount.

## Patentansprüche

1. Vernähinstrument (1), das einen in einen Körperhohlraum einzuführenden und mit einer hohlen Einstechnadel (13) mit einer an einem Ende ausgebildeten Öffnung ausgestatteten Einführabschnitt (10) und einen Bedienabschnitt (21) aufweist, der zur Bedienung eines Schiebebauteils (30) vorgesehen ist, das durch den Einführabschnitt hindurchgeführt ist, wobei das Schiebebauteil dazu dient, ein Ankerbauteil (16), das an einem Vernähmaterial (15) befestigt ist, aus dem Ende der Einstechnadel herauszuschieben, und das Vernähmaterial durch ein biologisches Gewebe hindurchzuführen ist, während das Ankerbauteil in dem biologischen Gewebe gehalten ist, und wobei der Bedienabschnitt umfasst:
einen Strömungseinführungsabschnitt (25) zur Einführung einer aus der Einstechnadel abzuführenden Strömung und **gekennzeichnet ist durch**
einen Strömungspfad (24), der diese Strömung innerhalb der Einstechnadel ermöglicht, wenn das Ankerbauteil aus der Einstechnadel geschoben werden kann.

2. Vernähinstrument nach Anspruch 1, wobei das Ankerbauteil mit einer zylindrischen Form ausgebildet ist.

3. Vernähinstrument nach Anspruch 1, wobei der Bedienabschnitt (20) einen mit dem Strömungseinführungsabschnitt ausgestatteten Hauptkörperabschnitt (74) und einen Nadelbedienabschnitt (76) aufweist, der bezüglich des Hauptkörperabschnitts bewegbar ist und die Einstechnadel vor- und zurückbewegen kann, und wobei der Nadelbedienabschnitt mit einer Verbindungsöffnung zur Herstellung einer Verbindung zwischen dem Strömungseinführungsabschnitt und dem Strömungspfad sowie mit Strömungsabsperrbauteilen ausgestattet ist, die die Strömung von dem Strömungseinführungsabschnitt zu dem Strömungspfad absperren, sobald die Einstechnadel um eine gegebene Strecke bewegt worden ist.

## Revendications

1. Instrument de suture (1) qui a une partie insérée (10) qui est destinée à être introduite dans une cavité corporelle et est équipée d'une aiguille de piquage creuse (13) ayant une ouverture formée à une extrémité ; et une partie opérationnelle (21), qui sert à faire fonctionner un élément de poussée (30) qui est fait passer à travers ladite partie insérée ; dans lequel ledit élément de poussée est employé pour pousser un élément d'ancrage (16) qui est fixé à un matériel de suture (15) hors de l'extrémité de ladite aiguille de piquage, et ledit matériel de suture est destiné à être fait passer à travers un tissu biologique, tandis que ledit élément d'ancrage est retenu dans ledit tissu biologique ; ladite partie opérationnelle comprenant :
une partie d'introduction de flux (25) pour introduire un flux destiné à être déchargé de ladite aiguille de piquage ; et **caractérisé par**
un chemin de flux (24) qui permet que ce flux à l'intérieur de ladite aiguille de piquage lorsque ledit élément d'ancrage est activé soit poussé hors de ladite aiguille de piquage.

2. Instrument de suture selon la revendication 1, dans lequel ledit élément d'ancrage est formé de manière à avoir une forme cylindrique.

3. Instrument de suture selon la revendication 1, dans lequel ladite partie opérationnelle (20) a une partie principale de corps (74) qui est équipée avec ladite partie d'introduction de flux, et une partie opérationnelle d'aiguille (76) qui est mobile par rapport à ladite partie principale de corps et peut faire avancer et rétracter ladite aiguille de piquage ; et dans lequel ladite partie opérationnelle d'aiguille est prévue avec un trou de communication pour une communication entre ladite partie d'introduction de flux et ledit chemin de flux, et des éléments d'isolement de flux pour couper le flux de ladite partie d'introduction de flux jusqu'audit chemin de flux, une fois que ladite aiguille de piquage a été déplacée d'une quantité fixée.
